# EUROPEAN PATENT APPLICATION

(11) **EP 4 520 522 A1**
(43) Date of publication of application: **12.03.2025**
(21) Application number: 23799473.6
(22) Date of filing: 28.04.2023
(51) Int. Cl.: B32B 9/00, A01N 25/00, A01N 25/34, A01N 59/16, A01P 3/00, B05D 3/10, B05D 5/00, B05D 7/24, C09D 1/00, C09D 5/14

(54) **ARTICLE HAVING ZINC OXIDE COATING FILM, METHOD FOR PRODUCING SAME, AND METHOD FOR DEODORIZING SAME**

(30) Priority: 06.05.2022 JP 2022076434; 21.09.2022 JP 2022150089
(71) Applicant: Tosoh Finechem Corporation, Shunan-shi, Yamaguchi 746-0006 (JP)
(72) Inventor: TOYOTA, Kouji, Shunan-shi, Yamaguchi 746-0006 (JP); MORI, Haruna, Shunan-shi, Yamaguchi 746-0006 (JP); INOUE, Takahiro, Shunan-shi, Yamaguchi 746-0006 (JP); AOKI, Masahiro, Shunan-shi, Yamaguchi 746-0006 (JP)
(74) Representative: f & e patent
(86) International application number: PCT/JP2023/016846
(87) International publication number: WO 2023/214543

(57) **Abstract**

Provided is an article having a zinc oxide coating film on a substrate, wherein a surface area increase ratio Sdr measured on the surface of the zinc oxide coating film is 2.00% or more and 13.00% or less, and an antibacterial activity value (in accordance with JIS Z 2801:2010) measured on the surface of the zinc oxide coating film is 2.0 or more.

## Description

### CROSS REFERENCE TO RELATED APPLICATIONS

This application claims priority to Japanese Patent Application No. 2022-076434 filed on May 6, 2022 and Japanese Patent Application No. 2022-150089 filed on September 21, 2022. Each of the above applications is hereby expressly incorporated by reference, in its entirety, into the present application.

### TECHNICAL FIELD

The present invention relates to an article having a zinc oxide coating film and a method of manufacturing the same, and a deodorizing method.

### BACKGROUND ART

Zinc oxide is also called zinc flower or zinc white, and is used in industrial applications, for example, as a white pigment. In addition, since fine particles of zinc oxide have an ultraviolet shielding effect and are transparent to visible light, zinc oxide is widely used in a variety of applications, such as cosmetics, sunscreens, pharmaceuticals, paints, and plastics (see, for example, PTL 1 to 8 (the entire disclosures of which are expressly incorporated herein by reference)).
PTL 1: Japanese Patent Application Publication No. 2003-95655
PTL 2: Japanese Examined Patent Publication No. H01-50202
PTL 3: Japanese Patent Application Publication No. 2018-115098
PTL 4: Japanese Patent Application Publication No. 2021-001278
PTL 5: Japanese Patent Application Publication No. H05-156510
PTL 6: WO 2013/073555
PTL 7: Japanese Patent Application Publication No. H09-286615
PTL 8: Japanese Patent Application Publication No. 2011-170979

### SUMMARY OF INVENTION

PTL 1 to 3 propose the use of zinc oxide, which has been imparted with strong water repellency by surface treatment with silicone, for cosmetic purposes.

Meanwhile, antibacterial activity of zinc oxide has been attracting attention in recent years. In this regard, PTL 4 to 7 propose using zinc oxide to impart antibacterial properties to articles.

Furthermore, PTL 8 discloses a method for forming a zinc oxide coating film on the surface of an article, in which the zinc oxide coating film is formed from a composition based on a partial hydrolysate of an organozinc compound.

In view of the above, one aspect of the present invention relates to an antibacterial article having a zinc oxide coating film.

One aspect of the present invention is as follows.
[1] An article having a zinc oxide coating film on a substrate, wherein
   a surface area increase ratio Sdr measured on the surface of the zinc oxide coating film is 2.00% or more and 13.00% or less, and an antibacterial activity value (in accordance with JIS Z 2801:2010) measured on the surface of the zinc oxide coating film is 2.0 or more.
[2] The article according to [1], in which an antiviral activity value (in accordance with ISO 21702) measured on the surface of the zinc oxide coating film is 2.0 or more.
[3] The article according to [1] or [2], in which a thickness of the zinc oxide coating film is 50 nm or more and 1000 nm or less.
[4] The article according to any one of [1] to [3], in which a transmittance of the zinc oxide coating film at a wavelength of 550 nm is 80.0% or more.
[5] The article according to any one of [1] to [4], in which the surface area increase ratio Sdr measured on the surface of the zinc oxide coating film is 2.00% or more and 9.00% or less.
[6] The article according to [1] or [2], in which a thickness of the zinc oxide coating film is 50 nm or more and 1000 nm or less, and a transmittance of the zinc oxide coating film at a wavelength of 550 nm is 80.0% or more.
[7] The article according to [6], in which the surface area increase ratio Sdr measured on the surface of the zinc oxide coating film is 2.00% or more and 9.00% or less.
[8] The article according to [1], in which an antiviral activity value (in accordance with ISO 21702) measured on the surface of the zinc oxide coating film is 2.0 or more,
   a thickness of the zinc oxide coating film is 50 nm or more and 1000 nm or less, and
   a transmittance of the zinc oxide coating film at a wavelength of 550 nm is 80.0% or more.
[9] The article according to [8], in which the surface area increase ratio Sdr measured on the surface of the zinc oxide coating film is 2.00% or more and 9.00% or less.
[10] The article according to any one of [1] to [9], which is a deodorant.
[11] A method of manufacturing the article according to any one of [1] to [10], the method including:
   forming the zinc oxide coating film by applying a coating liquid containing an organozinc compound represented by formula (1)

      Formula (1): R¹-Zn-R²

      (in formula (1), R¹ and R² each independently represent a linear or branched alkyl group having 1 to 8 carbon atoms)
   in an atmosphere in which water is present.
[12] The manufacturing method according to [11], in which the application is carried out under atmospheric pressure.
[13] The manufacturing method according to [11] or [12], in which the organozinc compound represented by the formula (1) is diethylzinc.
[14] A deodorizing method including placing the article according to [10] in an atmosphere containing a gas to be deodorized.
[15] The deodorizing method according to [14], in which the gas to be deodorized is selected from the group consisting of hydrogen sulfide gas, methyl mercaptan gas, and isovaleric acid gas.

According to one aspect of the present invention, an article imparted with antibacterial properties due to having a zinc oxide coating film can be provided. According to another aspect of the present invention, a manufacturing method of such an article can be provided. According to yet another aspect of the present invention, a deodorizing method using the article can be provided.

### DESCRIPTION OF EMBODIMENTS

### [Article Having Zinc Oxide Coating]

One aspect of the present invention relates to an article having a zinc oxide coating film on a substrate, wherein a surface area increase ratio Sdr measured on the surface of the zinc oxide coating film is 2.00% or more and 13.00% or less, and an antibacterial activity value (in accordance with JIS Z 2801:2010) measured on the surface of the zinc oxide coating film is 2.0 or more.

In the course of a comprehensive research of antibacterial articles having a zinc oxide coating film, the present inventors have found that there is a correlation between the surface structure of the zinc oxide coating film and antibacterial activity thereof. This is a new finding obtained by the present inventors, which is not described in the above-mentioned literature. As a result of additional diligent research, the present inventors newly found that a zinc oxide coating film having a surface area increase ratio Sdr of 2.00% or more measured on the surface can exhibit an antibacterial activity value of 2.0 or more. Furthermore, the present inventors have newly discovered that a zinc oxide coating film having a surface area increase ratio Sdr of 2.00% or more measured on the surface can exhibit an antiviral activity value of 2.0 or more. That is, according to one aspect of the present invention, an article having a zinc oxide coating film on a substrate can be provided, in which the surface area increase ratio Sdr measured on the surface of the zinc oxide coating film is 2.00% or more and 13.00% or less, and the antiviral activity value (in accordance with ISO 21702) measured on the surface of the zinc oxide coating film is 2.0 or more.

The above article will be described in more detail below.

### <Zinc Oxide Coating>

### (Surface Area Increase Ratio Sdr)

"Surface area increase ratio Sdr" is a surface property parameter defined in ISO25178-2:2012, and is generally also called "developed interfacial area ratio". It indicates how much the developed area (surface area) of a defined region has increased relative to the area of the defined region. More specifically, it expresses how much the surface area (developed area) of the surface of the zinc oxide coating film has increased relative to the surface area of a defined region, which is the surface of the substrate on which the zinc oxide coating film is provided. A completely flat surface has an Sdr of 0%. Measurements are performed using a laser microscope. A laser microscope is a non-contact microscope that uses a laser as a light source and can measure the unevenness of an object surface in three dimensions. Measurement conditions that can be used include, for example, the measurement conditions described in the Examples section hereinbelow. Hereinafter, "surface area increase ratio Sdr" will also be referred to simply as "Sdr".

From the viewpoint of improving antibacterial activity, the surface area increase ratio Sdr measured on the surface of the zinc oxide coating film located on the substrate of the above article is 2.00% or more, preferably 2.20% or more, and more preferably 2.40% or more, 2.60% or more, 2.80% or more, 3.00% or more, 3.20% or more, 3.40% or more, 3.60% or more, 3.80% or more, 4.00% or more, 4.20% or more, 4.40% or more, and 4.60% or more in that order. Also, from the viewpoint of improving antiviral activity, it is preferable that the surface area increase ratio Sdr measured on the surface of the zinc oxide coating film located on the substrate of the above article be in the above range.

There are various theories about the antibacterial mechanism of zinc oxide, and a mechanism according to which zinc ions destroy cell membranes, and a mechanism according to which hydrogen peroxide generated from the zinc oxide surface damages the DNA or enzymes of bacteria, killing them, have been considered.

The present inventors presume that the antibacterial effect due to hydrogen peroxide generation, more specifically, the antibacterial effect of hydroxyl radicals generated from hydrogen peroxide, is the greatest among these (see Journal of the Caeramic Society of Japan 106 [10] 1007-1011 (1998)). The present inventors also presume that the antiviral mechanism of zinc oxide is that viruses are killed as a result of active oxygen (for example, hydroxyl radicals generated from hydrogen peroxide) damaging the membranes of virus particles, called the envelopes.

Hydrogen peroxide is generated when the zinc oxide surface comes into contact with oxygen and/or water. Therefore, the present inventors presume that adjusting the surface structure of the zinc oxide coating film, particularly by increasing the value of Sdr, will increase the amount of hydrogen peroxide generated, thereby improving the antibacterial activity and, further, the antiviral effect. Previously, in order to enhance the antibacterial activity of zinc oxide, studies had been conducted only on controlling the particle size of zinc oxide particles or improving the specific surface area of zinc oxide particles (see Japanese Patent Application Publication No. 2021-001278 (PTL 4), Japanese Patent Application Publication No. H05-156510 (PTL 5), WO 2013/073555 (PTL 6), and Japanese Patent Application Publication No. H09-286615 (PTL 7)), but no proposals had been made regarding the surface structure of zinc oxide coating film located on the substrate of the article. Meanwhile, from the viewpoint of having a transparency making it possible to obtain a functional transparent coating on the substrate of the article, the surface area increase ratio Sdr is 13.00% or less, preferably 12.00% or less, more preferably 11.00% or less, and further preferably 10.00% or less, 9.00% or less, 8.80% or less, 8.60% or less, 8.40% or less, 8.20% or less, and 8.00% or less in that order. The means for controlling the Sdr will be described hereinbelow.

### (Antibacterial Activity Value)

The antibacterial activity value (in accordance with JIS Z 2801:2010) measured on the surface of the zinc oxide coating film located on the substrate of the article is 2.0 or more. An article exhibiting such an antibacterial activity value is useful as an antibacterial article in various applications.

In the present invention and present specification, the term "antibacterial activity value" refers to at least the antibacterial activity value against Escherichia coli and/or Staphylococcus aureus, which are the test bacteria described in JIS Z 2801:2010. The antibacterial activity value is an index value in accordance with the test method in JIS Z 2801:2010 "Antibacterial Products -- Test for Antibacterial Activity and Efficacy". In one embodiment, the antibacterial activity value measured on the surface of the zinc oxide coating film located on the substrate of the article in accordance with the test method in JIS Z 2801:2010 "Antibacterial Products -- Test for Antibacterial Activity and Efficacy" using methicillin-resistant Staphylococcus aureus as the test bacteria can be 2.0 or more. In one embodiment, the antibacterial activity value measured on the surface of the zinc oxide coating film located on the substrate of the article in accordance with the test method in JIS Z 2801:2010 "Antibacterial Products - Test for Antibacterial Activity and Efficacy" using Klebsiella pneumoniae as the test bacteria can be 2.0 or more. In one embodiment, the antibacterial activity value measured on the surface of the zinc oxide coating film located on the substrate of the article in accordance with the test method in JIS Z 2801:2010 "Antibacterial Products - Test for Antibacterial Activity and Efficacy" using Moraxella as the test bacteria can be 2.0 or more. In one embodiment, the antibacterial activity value measured on the surface of the zinc oxide coating film located on the substrate of the article in accordance with the test method in JIS Z 2801:2010 "Antibacterial Products - Test for Antibacterial Activity and Efficacy" using Pseudomonas aeruginosa as the test bacteria can be 2.0 or more.

The antibacterial activity value is determined as the difference in the average logarithm value of the number of live bacteria between an antibacterial treated product and an untreated product after inoculating the test bacteria and culturing them for 24 hours. If the antibacterial activity value determined in this way is 2.0 or more, the bacterial death rate can be said to be 99% or more, and the treatment is generally considered to have antibacterial activity. The above article has a coating containing zinc oxide having antibacterial activity on the substrate, and the Sdr of the surface is within the range described above, so that a high antibacterial activity value of 2.0 or more can be exhibited.

### (Antiviral Activity Value)

The antiviral activity value (based on ISO 21702) measured on the surface of the zinc oxide coating film located on the substrate of the article can be 2.0 or more. Articles that exhibit such an antiviral activity value are useful as antiviral articles in various applications.

In the present invention and present specification, the "antiviral activity value" refers to at least the antiviral activity value against the new coronavirus SARS-CoV-2 (COVID-19). The antiviral activity value is an index value based on the test method in ISO 21702 "Measurement of antiviral activity on plastics and other non-porous surfaces". The new coronavirus SARS-CoV-2 (COVID-19) is an enveloped virus. In one embodiment, the antiviral activity value measured on the surface of the zinc oxide coating film located on the substrate of the article in accordance with the test method in ISO 21702 "Measurement of antiviral activity on plastics and other non-porous surfaces" using enveloped viruses such as influenza virus, measles virus, rubella virus, AIDS virus, and herpes virus as test viruses can be 2.0 or more. The article can exhibit high antiviral effects against various enveloped viruses.

The antiviral activity value is determined as the difference in the average logarithm value of the number of live viruses between an antiviral treated product and an untreated product after inoculating the test viruses and culturing them for 24 hours. If the antiviral activity value determined in this way is 2.0 or more, the virus death rate can be said to be 99% or more, and the treatment is generally considered to have antiviral activity. The above article has a coating containing zinc oxide having antiviral activity on the substrate, and the Sdr of the surface is within the range described above, so that a high antiviral activity value of 2.0 or more can be exhibited.

### (Film Thickness)

The film thickness of the zinc oxide coating film can be adjusted appropriately depending on the application and is not particularly limited. For example, the film thickness of the zinc oxide coating film can be 50 nm or more, 100 nm or more, 150 nm or more, 200 nm or more, 250 nm or more, 300 nm or more, 350 nm or more, 400 nm or more, 450 nm or more, 500 nm or more, 550 nm or more, or 600 nm or more. The film thickness of the zinc oxide coating film can be, for example, 10 µm or less. From the viewpoint of transparency of the zinc oxide coating film, the film thickness is preferably 2000 nm or less, more preferably 1500 nm or less, and even more preferably 1000 nm or less. According to the research conducted by the present inventors, the Sdr value of the zinc oxide coating film tends to increase as the film thickness increases. Therefore, adjust the film thickness is an example of means for controlling Sdr. The film thickness can be measured by a stylus-type surface profiler. An example of a measuring device is the measuring device described in the Examples section below.

### (Transmittance)

From the viewpoint of the usefulness of the article in various applications, it is preferable that the zinc oxide coating film have high transparency. In this regard, it is preferable that the zinc oxide coating film have a high visible light transmittance. For example, the transmittance of the zinc oxide coating film is preferably 80.0% or more at a wavelength of 550 nm, more preferably 82.0% or more, even more preferably 85.0% or more, still more preferably 88.0% or more, and even more preferably 90.0% or more. The transmittance can be, for example, 100% or less, 99.0% or less, 98.0% or less, 97.0% or less, 96.0% or less, or 95.0% or less. The higher the transmittance, the higher the transparency, which is preferable. The transmittance can be measured by a commercially available spectrophotometer.

The above article can have the zinc oxide coating film on at least a portion of the substrate. In addition, the zinc oxide coating film is preferably located as the outermost layer of the article at the location where the coating film is provided. When the substrate has a front surface and a back surface, the article can have the zinc oxide coating film on, for example, a portion or the entire surface of either or both of the front surface and the back surface. In addition, the zinc oxide coating film may be provided on at least a portion or the entire surface of the side surface of the substrate. The substrate may be a substrate alone or may be a substrate having one or more layers provided thereon.

### <Substrate>

The substrate is not particularly limited as long as it is an article to which antibacterial and/or antiviral properties are to be imparted. In one embodiment, the substrate is preferably one on which the coating liquid described below can be easily applied.

The material constituting the substrate is not particularly limited, can be exemplified by metals, metal oxides, glass, concrete, various plastics, paper, wood, etc., and may be a composite material containing one or more of these.

The substrate may have a plate-like or curved shape and may have an uneven surface.

As a result of having the zinc oxide coating film on the substrate, the article can exhibit high antibacterial performance with an antibacterial activity value of 2.0 or more, and is useful as an article that can be called an antibacterial article, antibacterial processed article, antibacterial processed product, and the like. Furthermore, as a result of having the zinc oxide coating film on the substrate, the article can exhibit high antiviral performance with an antiviral activity value of 2.0 or more, and is useful as an article that can be called an antiviral article, antiviral processed article, antiviral processed product, and the like.

The article is not particularly limited and examples thereof include various articles that are required to have antibacterial and/or antiviral properties, such as installed in medical institutions, commercial facilities, schools, public facilities, food and pharmaceutical factories, research institutes, nursing care sites, cooking and food and beverage related sites, transportation facilities, and the like. Specific examples of such articles include doors, doorknobs, handrails, handles, faucets, glass, laboratory equipment, tables, chairs, home appliances, office supplies, medical equipment, push buttons, keyboards, computer mice, touch panels, mobile communication devices, vein and fingerprint authentication devices, operation buttons, switches, and the like.

The zinc oxide coating film of the article can preferably exhibit high transparency, which provides visibility, while zinc oxide also has the property of blocking ultraviolet rays. Therefore, the zinc oxide coating film can also contribute to suppressing deterioration of antibacterial and/or antiviral articles caused by ultraviolet rays.

Furthermore, as a result of the research conducted by the present inventors, it has become clear that the above article can exhibit a deodorizing effect against malodorous gases. For example, by placing the article in an atmosphere containing a gas to be deodorized, the article can function as a deodorizing material to reduce the concentration of the malodorous gas in the atmosphere. That is, one aspect of the present invention relates to a deodorizing method including placing the article in an atmosphere containing a gas to be deodorized. Examples of the malodorous gas to be deodorized include gases such as hydrogen sulfide, methyl mercaptan, and isovaleric acid. The atmosphere containing the gas to be deodorized may be a closed atmosphere or an open atmosphere. The volume, form, and malodorous gas concentration of the atmosphere containing the gas to be deodorized are not particularly limited.

### [Method of Manufacturing the Article]

One aspect of the present invention relates to a method of manufacturing the above article, the method including forming the zinc oxide coating film by applying a coating liquid containing an organozinc compound represented by the following formula (1).

Formula (1): R¹-Zn-R²

(in formula (1), R¹ and R² each independently represent a linear or branched alkyl group having 1 to 8 carbon atoms.)

In the present invention and present specification, the term "zinc oxide coating film" means a coating film containing zinc oxide. In the above manufacturing method, the zinc oxide coating film can be formed by hydrolysis of an organozinc compound represented by formula (1). In this method, the zinc oxide coating film can be formed without a binder, as opposed to a method of forming a zinc oxide coating film using a coating liquid containing zinc oxide particles and a binder. In addition, this method can also firmly attach zinc oxide to the substrate surface, which is the surface to be coated. Articles that require antibacterial properties may be sterilized, and steam sterilization (moist heat sterilization) or dry heat sterilization is generally used. In either sterilization process, heating to 130°C or higher is generally performed, so it is desirable for the zinc oxide coating film that is to undergo such sterilization to have high heat resistance. Compared to zinc oxide coating films formed using zinc oxide particles and a binder, the zinc oxide coating film formed by the above method is preferable from the standpoint of high heat resistance thereof. However, the article according to one aspect of the present invention described above in detail is not limited to those manufactured by the manufacturing method described below.

### <Components of the Coating Liquid>

### (Organozinc Compound Represented by Formula (1))

The organozinc compound is represented by the following formula (1).
Formula (1):

R¹-Zn-R² (1)

The formula (1) will be explained in more detail hereinbelow.

In formula (1), R¹ and R² each independently represent a linear or branched alkyl group having 1 to 8 carbon atoms. The organozinc compound represented by formula (1) is a dialkylzinc. In one embodiment, R¹ and R² represent the same alkyl group, and in another embodiment, R¹ and R² represent different alkyl groups.

Specific examples of the alkyl group include methyl group, ethyl group, propyl group, isopropyl group, butyl group, isobutyl group, sec-butyl group, tert-butyl group, pentyl group, isopentyl group, neopentyl group, tert-pentyl group, hexyl group, isohexyl group, sec-hexyl group, tert-hexyl group, 2-hexyl, heptyl group, 2-ethylhexyl group, and octyl group.

The compound represented by formula (1) preferably has an alkyl group having 1 to 3 carbon atoms as the alkyl group, and more preferably is diethylzinc (i.e., R¹ and R² are both ethyl groups) because of high availability thereof.

The concentration of the organozinc compound represented by formula (1) in the coating liquid is preferably 15% by mass or less, more preferably 14% by mass or less, and even more preferably 13% by mass or less, and 12% by mass or less, taking the total amount of the coating liquid as 100% by mass. The concentration is preferably 1% by mass or more, more preferably 2% by mass or more, and even more preferably 3% by mass or more.

The concentration of the organozinc compound represented by formula (1) in the coating liquid is preferably in the above range from the viewpoints of manufacturing a zinc oxide coating film having a sufficient film thickness to exhibit antibacterial and/or antiviral properties, manufacturing a transparent zinc oxide coating film, and suppressing nozzle clogging due to pot life and solvent evaporation. Furthermore, in the research conducted by the present inventors, it was found that the Sdr value of the zinc oxide coating film formed tends to increase when the concentration of the organozinc compound represented by formula (1) in the coating liquid is increased. Therefore, adjusting the concentration is an example of a means for controlling Sdr.

### (Solvent)

The coating liquid contains at least one compound represented by formula (1) and can usually further contain a solvent. The solvent is not particularly limited, and examples thereof include aliphatic hydrocarbon solvents such as pentane, hexane, heptane, octane, and petroleum ether, aromatic hydrocarbon solvents such as benzene, toluene, ethylbenzene, xylene, mesitylene, and cyclohexylbenzene, and ether solvents such as 1,2-diethoxyethane, diethyl ether, diisopropyl ether, glyme, diglyme, triglyme, dioxane, tetrahydrofuran, and anisole. In addition, only one type of solvent can be used, or two or more types can be mixed in any ratio. Considering the solubility of the organozinc compound and the volatility of the solvent, xylene, mesitylene, and the like are the preferred solvents.

The coating liquid can contain only the above components, or can contain one or more known components in a freely selected ratio.

### <Application of Coating Liquid>

The coating liquid is applied in an atmosphere in which water is present. The relative humidity of the "atmosphere in which water is present" can be, for example, 20% or more and 100% or less. The "atmosphere in which water is present" can be, for example, an atmosphere with water content equivalent to a relative humidity of 20% to 100%. Alternatively, the "atmosphere in which water is present" can be an atmosphere of a mixed gas in which nitrogen and water are mixed, instead of the air atmosphere. The relative humidity of the "atmosphere in which water is present" is preferably 40% or more and 100% or less from the viewpoint of smooth generation of the zinc oxide coating film, and is preferably 50% or more and 100% or less, or 50% or more and 90% or less from the viewpoint of increasing the value of Sdr. Adjusting the relative humidity of the atmosphere in which coating is performed is one example of a means for controlling Sdr. For example, the research conducted by the present inventors has confirmed that the value of Sdr of the zinc oxide coating film formed can increase with a higher relative humidity of the atmosphere in which coating is performed.

The zinc oxide coating film can be formed on the substrate surface by applying the coating liquid to the substrate surface. Details of the substrate are as described above. The coating liquid can be applied by known methods such as spray coating, electrostatic coating, spin coating, dip coating, and the like. Spray coating is preferred because no limitation is placed on the shape of the substrate. With regard to the control of Sdr, the research conducted by the present inventors has confirmed that the Sdr value of the zinc oxide coating film formed may increase when the spray pressure in spray coating is reduced. Therefore, adjusting the spray pressure in spray coating is one example of a means for controlling Sdr. For example, the spray pressure can be a set value in the spray coating device used. The spray pressure may be set according to the specifications and the like of the spray coating device and is not particularly limited. In addition, the zinc oxide coating film can be thickened by repeating spray coating two or more times.

The spray coating will be described in detail below, but the present invention is not limited to the following description.

Spray coating on the substrate surface can be performed under atmospheric pressure or under pressure, preferably under atmospheric pressure, in an atmosphere in which water is present. Performing under atmospheric pressure is preferable because the equipment is easy to use. As the spray coating device, a commercially available spray coating device or a spray coating device with a known configuration can be used. The spray coating devices are of a movable nozzle type and a fixed nozzle type. In the movable nozzle type spray coating device, the nozzle moves in the X direction and Y direction during spray coating. The research conducted by the present inventors has shown that when the movable nozzle type spray coating device is used, the Sdr value of the zinc oxide coating film formed tends to be larger than when the fixed nozzle type spray coating device is used.

Spray coating on the substrate surface can be performed by heating the substrate. The substrate temperature during spray coating can be, for example, 350°C or less, and the substrate temperature can be controlled by a known heating means such as a heater. In the present invention and present specification, the "substrate temperature" refers to the temperature of the substrate surface to which the coating liquid is applied. If necessary, the substrate temperature can be set to a predetermined temperature, the solvent can be dried, and then heating can be performed at a predetermined temperature to form zinc oxide. The solvent drying temperature and the substrate temperature for the subsequent zinc oxide formation can be set to the same temperature, and the solvent drying and zinc oxide formation can be performed simultaneously. The substrate temperature can also be set, as appropriate, for example, by raising the temperature in multiple stages depending on the type of solvent contained in the coating liquid.

The ambient temperature of the atmosphere in which spray coating is performed can be, for example, 50°C or lower. It is preferable that spray coating be performed with an ambient temperature of 50°C or lower and a substrate temperature of 300°C or lower. From the viewpoint of promoting the crystallization of zinc oxide in the zinc oxide coating film, it is preferable that the ambient temperature at which spray coating is performed be 0°C or higher and 40°C or lower, and the substrate temperature be 100°C or higher and 250°C or lower. Furthermore, a cycle of "coating-drying-heating" can be performed once or twice or more times. By repeating the cycle twice or more, the zinc oxide coating film can be thickened. The amount of the coating liquid to be applied can be adjusted depending on the concentration of the organozinc compound in the coating liquid, the specifications of the spray coating device to be used, the thickness of the zinc oxide coating film to be formed, and the like.

### EXAMPLES

The present invention will be described below based on Examples. However, the present invention is not limited to the embodiments shown in Examples.

The physical properties of the zinc oxide coating film described below were measured using the following measuring devices under the following measurement conditions.

The surface area increase ratio Sdr was measured using a VK-9500/VK-9510 laser microscope (manufactured by Keyence Corporation) under the following measurement conditions.
Measurement conditions: lens 20x (field of view: 700 µm × 530 µm)
Z-direction step: 0.01 µm

The film thickness was measured using a stylus surface profiler (DektakXT-S, manufactured by Bruker Nano Corp.).

The transmittance was measured using a spectrophotometer (manufactured by JASCO Corporation) to determine the transmittance at a wavelength of 550 nm.

The spray coating device A described below is a rCoater (movable nozzle type) manufactured by Asahi Sunac Corporation. The spray coating device B described below is a spray coating device (fixed nozzle type) described in Japanese Patent Application Publication No. 2011-170979.

In Table 1, the "Spray pressure" column indicates the spray pressure set when using the spray coating device A. The spray coating device B does not have a spray pressure control unit and controls the flow rate of the entrained gas during spray coating. Therefore, in Table 1, "N/A" (Not available) is indicated in the "Spray pressure" column for Example 3 in which the spray coating device B was used.

### [Example 1]

### <Preparation of Coating Liquid A>

A total of 48.25 g of diethylzinc was added to 296.40 g of xylene. Coating liquid A was obtained by thorough stirring.

The coating liquid A was prepared under a nitrogen gas atmosphere, and the solvent (xylene) was dehydrated and degassed before use.

### <Application of Coating Liquid>

A glass substrate (Eagle XG, manufactured by Corning Inc.) measuring 5 cm × 5 cm was placed on a substrate holder and heated to a substrate temperature of 200°C. Then, 1 mL of coating liquid A was spray-coated (number of spray coatings: 1) using spray coating device A (spray pressure: see Table 1) in the air in which water was present at atmospheric pressure, an atmosphere temperature of 25°C, and a relative humidity of 50%.

As a result, an article having a zinc oxide coating film on one surface of the substrate was obtained.

### [Example 2]

An article having a zinc oxide coating film on one surface of the substrate was obtained by the method described for Example 1, except that the relative humidity of the atmosphere in which the spray coating was performed was changed as shown in Table 1.

### [Example 3]

### <Preparation of Coating Liquid B>

A total of 18.14 g of diethylzinc was added to 163.08 g of diisopropyl ether. Coating liquid B was obtained by thorough stirring.

The coating liquid B was prepared under a nitrogen gas atmosphere, and the solvent (diisopropyl ether) was dehydrated and degassed before use.

### <Application of Coating Liquid>

A glass substrate (Eagle XG, manufactured by Corning Inc.) measuring 5 cm × 5 cm was placed on a substrate holder and heated to a substrate temperature of 200°C. Then, 16 mL of coating liquid B was spray-coated (number of spray coatings: 1) using spray coating device B in the air in which water was present at atmospheric pressure, an atmosphere temperature of 25°C, and a relative humidity of 80%.

As a result, an article having a zinc oxide coating film on one surface of the substrate was obtained.

### [Example 4]

### <Preparation of Coating Liquid C>

A total of 30.05 g of diethylzinc was added to 270.45 g of xylene. Coating liquid C was obtained by thorough stirring.

The coating liquid C was prepared under a nitrogen gas atmosphere, and the solvent (xylene) was dehydrated and degassed before use.

### <Application of Coating Liquid>

A glass substrate (Eagle XG, manufactured by Corning Inc.) measuring 5 cm × 5 cm was placed on a substrate holder and heated to a substrate temperature of 200°C. Then, 1 mL of coating liquid C was sprayed once using spray coating device A (spray pressure: see Table 1) in the air in which water was present at atmospheric pressure, an atmosphere temperature of 25°C, and a relative humidity of 50%. This spray coating was repeated a total of five times, and a total of 5 mL of coating liquid C was spray-coated.

As a result, an article having a zinc oxide coating film on one surface of the substrate was obtained.

### [Example 5]

An article having a zinc oxide coating film on one surface of the substrate was obtained by the method described for Example 4, except that the relative humidity of the atmosphere in which the spray coating was performed was changed as shown in Table 1.

### [Example 6]

A glass substrate (Eagle XG, manufactured by Corning Inc.) measuring 5 cm × 5 cm was placed on a substrate holder and heated to a substrate temperature of 200°C. Then, 1 mL of the coating liquid C prepared as described above was sprayed once onto one surface of the substrate using spray coating device A (spray pressure: see Table 1) in the air in which water was present under atmospheric pressure, an atmosphere temperature of 25°C, and a relative humidity shown in Table 1. This spray coating was repeated a total of eight times, and a total of 8 mL of coating liquid C was spray-coated.

As a result, an article having a zinc oxide coating film on one surface of the substrate was obtained.

### [Comparative Example 1]

An article having a zinc oxide coating film on one surface of the substrate was obtained by the method described in Example 6, except that coating liquid A prepared as described above was used.

### [Comparative Example 2]

An article having a zinc oxide coating film on one surface of the substrate was obtained by the method described in Example 4, except that the relative humidity of the atmosphere in which the spray coating was performed was changed as shown in Table 1, and the spray pressure was set to the value shown in Table 1.

### [Antibacterial Test]

Antibacterial tests of each of the articles obtained in Examples 1 to 6 and Comparative Example 2 were conducted in the Japan Food Research Laboratories. The antibacterial tests were conducted by the following method.

The antibacterial activity value of the zinc oxide coating film surface of each article (i.e., the outermost surface of the article) was determined by the above-described method in accordance with JIS Z 2801:2010 using Escherichia coli and Staphylococcus aureus as test bacteria.

For articles of Examples 1, 2, and 4, antibacterial tests were also conducted by the above-described method in accordance with JIS Z 2801:2010 using methicillin-resistant Staphylococcus aureus as test bacteria. In the antibacterial tests, the above-mentioned glass substrate (without coating) was used as the untreated product.

For articles of Example 4, antibacterial tests were also conducted by the above-described method in accordance with JIS Z 2801:2010 using Klebsiella pneumoniae, Moraxella, and Pseudomonas aeruginosa as test bacteria. In the antibacterial tests, the above-mentioned glass substrate (without coating) was used as the untreated product.

As shown in Table 2, in all of Examples 1 to 6, the antibacterial activity values against Escherichia coli and Staphylococcus aureus of 2.0 or more, which is considered to indicate antibacterial activity, were obtained as test results in the antibacterial tests. In Comparative Example 2, the antibacterial activity values against Escherichia coli and Staphylococcus aureus were also 2.0 or more.

In Examples 1, 2, and 4, antibacterial activity values against methicillin-resistant Staphylococcus aureus of 2.0 or more, which is considered to indicate antibacterial activity, were also obtained.

In Example 4, antibacterial activity values against Klebsiella pneumoniae, Moraxella, and Pseudomonas aeruginosa of 2.0 or more, which is considered to indicate antibacterial activity, were also obtained.

In Comparative Example 1, the transmittance of the zinc oxide coating film at a wavelength of 550 nm was low and the transparency degraded. Therefore, the antibacterial test was not conducted.

The above results are shown in the table below.

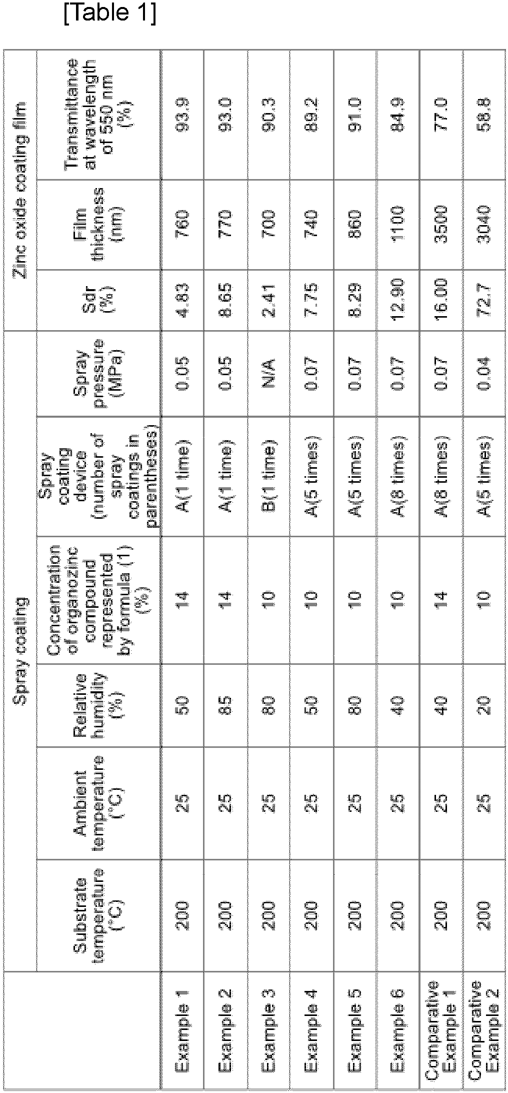

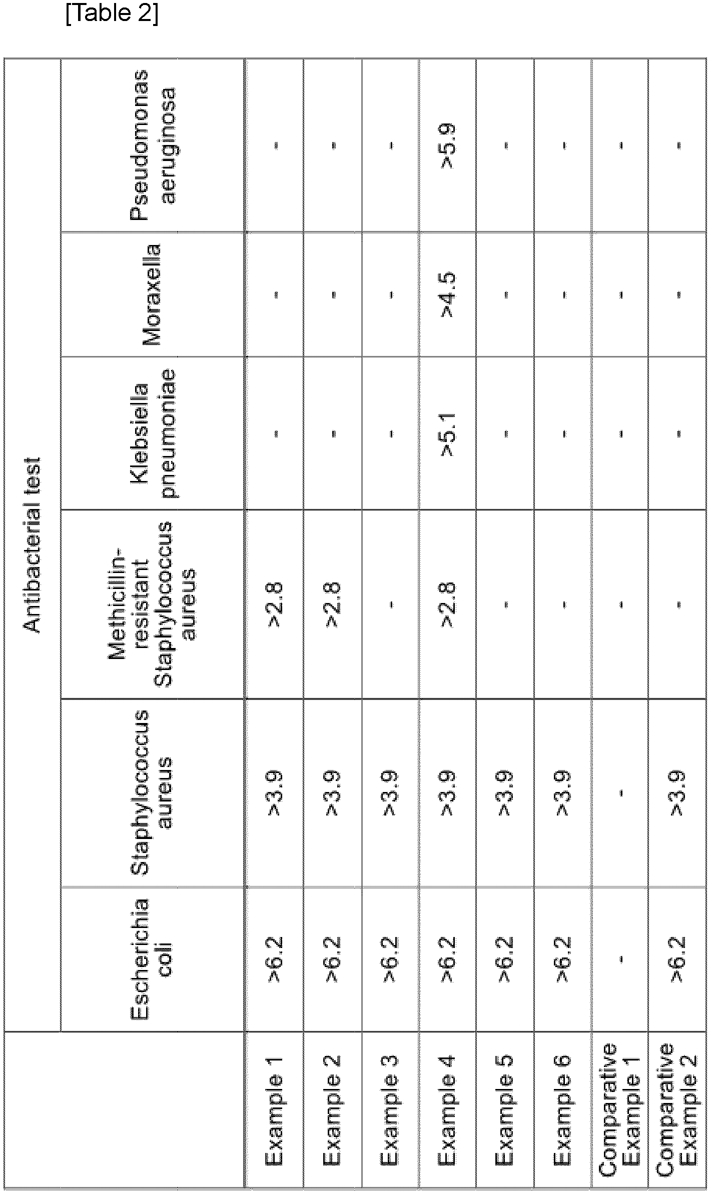

### [Antiviral Test]

The antiviral test of the article of Example 4 was carried out at the Japan Textile Products Quality and Technology Center (Kobe Testing Center), which is a general incorporated foundation. The antiviral test against SARS-CoV-2 mutant strain (Omicron strain) was carried out by the following method in accordance with ISO 21702. The above-mentioned glass substrate (without coating) was used as the untreated product.

In Example 4, the antiviral test result showed an antiviral activity value against SARS-CoV-2 mutant strain (Omicron strain) of 2.0 or more (specifically, "≥4.0") which is considered to indicate antiviral activity.
(1) The test specimens (the article of Example 4 and the untreated product) are placed on the bottom of a sterilized petri dish, and 0.4 mL of a test virus suspension prepared to a concentration of 1 × 10⁷ PFU/mL to 5 × 10⁷ PFU/mL is inoculated. The article of Example 4 is placed on the bottom of a petri dish with the zinc oxide coating film surface facing up.
(2) An adhesive film (40 mm × 40 mm) is covered and pressed lightly so that the test virus suspension is spread over the entire film.
(3) The petri dish is covered with a lid.
(4) The covered petri dish is allowed to stand under conditions of an operating temperature of 25°C and a relative humidity of 90% or more for 24 hours, and then 10 mL of washout solution (a solution in which SCDLP is diluted 10-fold (by volume) with DMEM (Dulbecco's Modified Eagle's Medium) containing 2% by mass FBS (Fetal Bovine Serum)) is added to each test specimen.
(5) The surface of each test specimen and the adhesive film is rubbed to wash out the virus.
(6) The virus infectivity in the washout solution is measured using a plaque assay method. The host cells used are VeroE6/TMPRSS2 (cells derived from African green monkey kidneys).
(7) The above steps (1) to (6) are performed three times, and the virus infectivity is calculated.

### [Deodorizing Effect Test]

The deodorizing effect test of the article of Example 4 was carried out at the Japan Food Research Laboratories, a general incorporated foundation. The deodorizing effect test was carried out for each of the three malodorous gases, namely, hydrogen sulfide, methyl mercaptan, and isovaleric acid, by the following method. The unit "ppm" described below is based on volume.

As a sample, the article of Example 4 was placed in a smell bag (made of polyvinyl fluoride), sealed by heat-sealing. Then, 3 L of air was enclosed, and the gas to be tested was added so that the initial gas concentration became the set gas concentration.

The smell bag was allowed to stand at room temperature, and the concentration of the gas under testing in the smell bag was measured with a gas detector tube at each elapsed time. The above glass substrate (without coating film) was used as a control, and a blank test was also conducted without using a specimen or control.

As a result, in the test using the article of Example 4 as the specimen, as shown in Table 3, when hydrogen sulfide gas was used as the gas to be tested, the hydrogen sulfide gas, which had an initial gas concentration of approximately 20 ppm, reached a concentration of 8 ppm (removal rate of 60%) after 24 hours.

Furthermore, when methyl mercaptan gas was used as the gas to be tested, as shown in Table 4, the methyl mercaptan gas, which had an initial gas concentration of approximately 8.0 ppm, reached a concentration of 2.1 ppm (removal rate of 74%) after 24 hours.

Furthermore, when isovaleric acid gas was used as the gas to be tested, as shown in Table 5, the isovaleric acid gas, which had an initial gas concentration of approximately 15 ppm, reached a concentration of 2.4 ppm (removal rate of 84%) after 24 hours.

**[Table 3]**

| Sample Category | Time elapsed | | | | |
|---|---|---|---|---|---|
| | 10 min | 1 h | 3 h | 6 h | 24 h |
| Specimen1) | 18 | 16 | 14 | 12 | 8 |
| | | | | | |
| Control | 20 | 20 | 20 | 20 | 20 |
| Blank test | 20 | 20 | 20 | 20 | 20 |

| | | | | | |
|---|---|---|---|---|---|
| Initial gas concentration: about 20 ppm | | | | | |

**[Table 4]**

| Sample Category | Time elapsed | | | | |
|---|---|---|---|---|---|
| | 10 min | 1 h | 3 h | 6 h | 24 h |
| Specimen1) | 8. 0 | 7. 4 | 7. 2 | 6. 0 | 2. 1 |
| | | | | | |
| Control | 8. 0 | 8. 0 | 8. 0 | 8. 0 | 8. 0 |
| Blank test | 8. 0 | 8. 0 | 8. 0 | 8. 0 | 8. 0 |

| | | | | | |
|---|---|---|---|---|---|
| Initial gas concentration: about 8.0 ppm | | | | | |

**[Table 5]**

| Sample Category | Time elapsed | | | | |
|---|---|---|---|---|---|
| | 10min | 1h | 3h | 6h | 24h |
| Specimen | 12 | 11 | 8. 4 | 5. 3 | 2. 4 |
| Control | 13 | 13 | 12 | 12 | 7. 5 |
| Blank test | 15 | 15 | 15 | 15 | 9. 8 |

| | | | | | |
|---|---|---|---|---|---|
| Initial gas concentration: about 15 ppm | | | | | |

The above results confirmed that the article of Example 4 was able to effectively deodorize various malodorous gases, as compared to the control sample and the blank test sample.

The articles according to one aspect of the present invention can be used as various articles that are required to have antibacterial and/or antiviral properties, such as installed in medical institutions, commercial facilities, public facilities, food and pharmaceutical factories, research institutions, nursing care sites, cooking and food and beverage related sites, transportation facilities, and the like. Furthermore, the article according to one aspect of the present invention can be used as a deodorizing material.

## Claims

1. An article having a zinc oxide coating film on a substrate,
wherein a surface area increase ratio Sdr measured on the surface of the zinc oxide coating film is 2.00% or more and 13.00% or less, and an antibacterial activity value (in accordance with JIS Z 2801:2010) measured on the surface of the zinc oxide coating film is 2.0 or more.

2. The article according to claim 1,
wherein an antiviral activity value (in accordance with ISO 21702) measured on the surface of the zinc oxide coating film is 2.0 or more.

3. The article according to claim 1,
wherein a thickness of the zinc oxide coating film is 50 nm or more and 1000 nm or less.

4. The article according to claim 1,
wherein a transmittance of the zinc oxide coating film at a wavelength of 550 nm is 80.0% or more.

5. The article according to claim 1,
wherein the surface area increase ratio Sdr measured on the surface of the zinc oxide coating film is 2.00% or more and 9.00% or less.

6. The article according to claim 1,
wherein a thickness of the zinc oxide coating film is 50 nm or more and 1000 nm or less, and a transmittance of the zinc oxide coating film at a wavelength of 550 nm is 80.0% or more.

7. The article according to claim 6,
wherein the surface area increase ratio Sdr measured on the surface of the zinc oxide coating film is 2.00% or more and 9.00% or less.

8. The article according to claim 1,
wherein an antiviral activity value (in accordance with ISO 21702) measured on the surface of the zinc oxide coating film is 2.0 or more,
a thickness of the zinc oxide coating film is 50 nm or more and 1000 nm or less, and
a transmittance of the zinc oxide coating film at a wavelength of 550 nm is 80.0% or more.

9. The article according to claim 8,
wherein the surface area increase ratio Sdr measured on the surface of the zinc oxide coating film is 2.00% or more and 9.00% or less.

10. The article according to any one of claims 1 to 9,
which is a deodorant.

11. A method of manufacturing the article according to any one of claims 1 to 9, the method comprising:
forming the zinc oxide coating film by applying a coating liquid comprising an organozinc compound represented by formula (1)
Formula (1): R¹-Zn-R²
(in formula (1), R¹ and R² each independently represent a linear or branched alkyl group having 1 to 8 carbon atoms)
in an atmosphere in which water is present.

12. The manufacturing method according to claim 11,
wherein the application is carried out under atmospheric pressure.

13. The manufacturing method according to claim 11,
wherein the organozinc compound represented by the formula (1) is diethylzinc.

14. A deodorizing method, comprising placing the article according to claim 10 in an atmosphere containing a gas to be deodorized.

15. The deodorizing method according to claim 14,
wherein the gas to be deodorized is selected from the group consisting of hydrogen sulfide gas, methyl mercaptan gas, and isovaleric acid gas.
